# EUROPEAN PATENT SPECIFICATION

(11) **EP 4 374 781 B1**
(45) Date of publication and mention of the grant of the patent: **25.02.2026**
(21) Application number: 22209176.1
(22) Date of filing: 23.11.2022
(51) Int. Cl.: A61B 5/0255, A61B 5/01, A61B 5/021, A61B 5/332, A61B 5/333, A61B 5/11, A61B 5/1455, G16H 50/20

(54) **BIOSIGNAL MONITORING SYSTEM**
BIOSIGNALÜBERWACHUNGSSYSTEM
SYSTÈME DE SURVEILLANCE DE BIOSIGNAL

(43) Date of publication of application: 29.05.2024
(73) Proprietor: Nokia Technologies Oy, 02610 Espoo (FI)
(72) Inventor: JANG, Si Young, 06182 Seoul (KR); FERLINI, Andrea, Cambridge, CB4 3QF (GB); QENDRO, Lorena, Cambridge, CB1 8DZ (GB)
(74) Representative: Sayer, Robert David

(56) References cited:
- EP-A1- 3 494 868
- US-A1- 2022 257 131

## Description

### Field

Example embodiments relate to a system for monitoring biosignals. Example embodiments also relate to a patch and an apparatus which may comprise part of the system.

### Background

Apparatuses are known for monitoring biosignals. A biosignal may be any signal in living beings that can be measured and monitored, e.g. for analysis and/or display purposes. For example, certain smartwatches may comprise sensors for placement next to a user's skin. The sensors may measure biosignals such as heart rate, blood oxygen saturation and/or body temperature. Additionally, smartwatches may use sensors such as a gyroscope and accelerometer within a so-called inertial measurement unit (IMU) for estimating force-related parameters such as number of steps taken in a particular time frame. The one or more biosignals and possibly the force-related parameters may be displayed on a screen of the smartwatch, usually within a health-monitoring application. US2022/257131 discloses a portable sensor system with measuring patch. EP3494868 discloses a system for sensing a parameter of a living being.

### Summary

The scope of protection of the invention is set out by the claims. The embodiments and features, if any, described in this specification that do not fall under the scope of the independent claim are to be interpreted as examples useful for understanding various embodiments of the invention.

According to the invention, there is provided a system according to claim 1.

At least one of the one or more first sensors may be configured to sense a different type of biosignal than any of the one or more second sensors. The apparatus may be configured to analyse and/or display the first biosignal data and the second biosignal data comprising the different types of biosignal.

The patch may have a substantially central aperture which aligns with the one or more second sensors when the apparatus is worn over the patch. The watch apparatus may comprise, for example, a substantially circular portion and the patch may be substantially ring-shaped. The substantially circular portion may comprise the one or more second sensors. Other shaped portions and patch apertures may be used.

The patch may further comprise a rechargeable energy storage means for providing power to the one or more first sensors and/or the transmitter and wherein the apparatus may be further configured to emit a wireless signal for recharging the energy storage means when the apparatus is worn over the patch.

The apparatus may further comprise a transmitter for transmitting an interrogation message to the patch, and the patch may further comprise a memory for storing the first biosignal data, and a transmitter for wirelessly transmitting, responsive to receiving the interrogation message, the stored first biosignal data to the apparatus.

The apparatus may be configured to transmit the interrogation message responsive to detecting the patch is in proximity to the apparatus. The patch may be configured to wirelessly transmit the first biosignal data to the second apparatus using an radiofrequency identification (RFID) or Bluetooth response message.

The apparatus may comprise one or more machined-learned models for performing analysis of one or both of the first biosignal data and the second biosignal data.

The apparatus may be further configured to transmit, to a remote second apparatus, one or both of the first and second biosignal data responsive to detecting a predetermined condition, for analysis by one or more machined-learned models at the second apparatus. The remote second apparatus may, for example, be a computing device which is paired with the apparatus. The predetermined condition may comprise one or more of: a charge level of a battery of the apparatus being below a predetermined threshold; and available processing resources of the apparatus being below a predetermined threshold.

The first biosignal data may represents one or more of heart rate, blood glucose level, perspiration and sodium level.

The second biosignal data may represent one or more of heart rate, blood pressure, electrocardiogram activity, temperature, and blood oxygen saturation.

The apparatus may comprise a watch apparatus, e.g. a smartwatch.

### Drawings

Embodiments will be described, by way of non-limiting example, with reference to the accompanying drawings as follows.
FIG. 1 shows a system according to one or more example embodiments.
FIG. 2 is a plan view of a patch, according to one or more example embodiments, which may comprise part of the FIG. 1 system.
FIGs. 3A and 3B are respective top and bottom plan views of an apparatus, according to one or more embodiments, which may comprise part of the FIG. 1 system.
FIG. 4 is a plan view of an alternative patch, according to one or more example embodiments, which may comprise part of the FIG. 1 system.
FIGs. 5A and 5B are respective bottom and top plan views of the apparatus when worn or placed over the patch, according to one or more example embodiments.
FIG. 6A is a schematic view of functional components of a patch according to one or more example embodiments.
FIG. 6B is a schematic view of functional components of an apparatus according to one or more example embodiments.
FIG. 6C is a schematic view of functional components of a further apparatus according to one or more example embodiments.
FIG. 7 is a flow diagram showing operations that may be performed by the patch and apparatus according to one or more example embodiments.
FIG. 8 is a schematic block diagram of an apparatus according to one or more example embodiments.
FIG. 9 is a non-transitory medium for storing computer-readable instructions which, when executed or processed by one or more processors of an apparatus, may perform operations according to one or more example embodiments.

### Detailed Description

Example embodiments relate to a system for monitoring biosignals. Example embodiments also relate to a patch and an apparatus forming part of the system. The apparatus may be a smartwatch, but other types of apparatus may also be used.

As described herein, a biosignal may be any signal in living beings that can be measured and monitored, e.g. for analysis and/or display purposes.

As mentioned in the background, smartwatches may comprise one or more sensors for measuring biosignals.

Sometimes, however, users may not wish to wear a smartwatch, or similar, due to its bulkiness, particularly at certain times such as when they sleep, swim or perform other forms of exercise or activity. This may lead to a loss of informative data which may otherwise provide useful health-related information for analysis and/or display purposes.

According to some example embodiments, there may be provided a system comprising at least two elements that, depending on a mode of operation, may cooperatively measure one or more biosignals at the same time or at different times.

For example, the system may comprise a patch which comprises a surface for attachment to a user, one or more first sensors for sensing respective first biosignals of the user and a transmitter for wirelessly transmitting to a remote apparatus first biosignal data representing the first biosignals of the user.

For example, the system may also comprise an apparatus, hereafter referred to as the "first apparatus" for being worn or placed over the patch. The first apparatus may comprise one or more second sensors at or near a lower surface for sensing respective second biosignals of the user and providing, e.g. generating, second biosignal data representing the second biosignals. The system may also comprise a receiver for wirelessly receiving the first biosignal data from the patch mentioned above.

The first apparatus may also be configured to analyse and/or display one or both of the first and second biosignal data.

Analysis may involve determining indications of a health status of the user based on, at least in part, the first and/or second biosignal data. For example, it may be determined that a user's heart rate and blood pressure is above a safe level for the user's age. An alert may be generated in response to such a determination. Other, more complex analysis operations may be performed on any number or combination of first and/or second biosignal data types, possibly involving the use of one or more machine learned (ML) models trained to classify particular combinations of input data, including biosignal data, as indicative of one or more health conditions. Such ML models may be trained using anonymised biosignal data provided by a plurality of other users.

In a first mode of operation, only the patch may be worn by the user. The patch may nevertheless measure and store first biosignal data using its one or more first sensors. The stored first biosignal data may also comprise timestamps or similar data indicative of when the measurements were taken.

In a second mode of operation, only the first apparatus may be worn or otherwise carried by the user. The first apparatus may nevertheless measure and store second biosignal data using its one or more second sensors. The stored second biosignal data may also comprise timestamps or similar data indicative of when the measurements were taken. The first apparatus may also provide the above-mentioned health monitoring application for analysis and/or display of the second biosignal data.

In a third mode of operation, the first apparatus may be worn or placed over the patch. In this way, the stored first biosignal data from the patch may be received by the first apparatus. Where both the first and second biosignal data represent, at least in part, a common type of biosignal, e.g. heart rate, then the received first biosignal data may fill-in temporal gaps in the second biosignal data for more accurate analysis. Where the first biosignal data represents, at least in part, one or more different types of biosignal than that of the second biosignal data, then the received first biosignal data adds different measurements to what is already being measured by the one or more second sensors.

According to some example embodiments, the patch may comprise one or more recesses or apertures for alignment with the one or more second sensors of the first apparatus when the first apparatus is worn or placed over the patch. In this way, the user's skin is exposed to the one or more second sensors because the patch does not block measurements by the one or more second sensors. The first apparatus can retain the patch in place on the user's body when both are worn for more efficient and reliable measurements.

In the above context, a patch may comprise a relatively thin (typically 0.5 - 2 mm in thickness) and flexible material, much like a sticking plaster, configured to be adhered to human skin by means of an attachment surface.

The attachment surface may comprise an adhering layer formed of a suitable adhesive. The adhering layer may be covered, over its outer surface, with a non-stick liner which protects against contaminants and particles. The user may remove the liner prior to placement on their skin. The flexible nature of the patch means that it may, in part, wrap-around part of the user's wrist or other body part.

The user may be provided with a plurality of patches that may be interchanged over time, for example if a patch loses its adhering properties and/or because different patches provide different first sensors or combinations of first sensors for measuring respective biosignals or combinations thereof.

In some cases, the adhering layer may be comprised of, at least in part, a hydrogel material. Hydrogels are water-insoluble, three-dimensional networks of polymer chains that may prevent irritation and/or allergic reactions with the skin. Hydrogels may also improve biosignal measurements.

The one or more first sensors provided on the patch may be of any suitable type.

For example, the one or more first sensors may be configured to sense one or more of heart rate, blood pressure, blood glucose level, perspiration and sodium level. This list is non-exhaustive. The sensors may comprise electrical sensors and/or chemical sensors. For example, for heart rate, the patch may comprise two electrocardiography (ECG) electrodes. For blood pressure, the patch may comprise two ultrasound transducers for sensing echoes from emitted ultrasounds that may be translated into blood pressure readings. For blood glucose level, invasive or non-invasive chemical sensors may be used. Invasive sensors may comprise a plurality of micro-needles that enter the skin and draw-out small amounts of glucose. Non-invasive sensors may detect glucose in other secretions, such as in cells from hair follicles. Perspiration and sodium levels may be detected using similar chemical sensors.

The sensed first biosignals may be converted into data form, stored and transmitted as first biosignal data to an external device, such as the first apparatus.

Electrical sensors, and other electrical or electronic components on the patch, may require a source of power from an on-board energy storage means, such as a rechargeable battery or from an energy harvesting means. An energy harvesting means may derive energy from one or more external sources, such as from solar energy cells and/or kinetic energy due to movement of the user. In the case of a rechargeable energy storage means, such as a rechargeable battery, the first apparatus may be configured to emit a wireless signal for recharging the energy storage means when worn or placed over the patch. The first apparatus, may, for example, issue a recharging signal when it detects that it is in proximity to the patch, which signal may be continuous or periodic and, optionally, may only be issued when the first apparatus senses that the level of the rechargeable battery is below a certain threshold.

The transmitter of the patch may comprise any form of transmitting means, for example a radio frequency identification (RFID) transmitter and/or a Bluetooth and/or Zigbee transmitter. This list in non-exhaustive.

The first apparatus may be a wearable apparatus, such as a smartwatch. The smartwatch may be an off-the-shelf (OTS) smartwatch configured or updated via suitable firmware and/or software to work co-operatively with the patch. The smartwatch may have a strap or band for locating the smartwatch over part of a user's body, for example over the user's wrist.

Alternatively, the first apparatus may comprise something other than a smartwatch, such as another form of wearable device or even a smartphone or tablet computer having one or more second sensors for measuring biosignals by exposing the second sensors to a user's skin. For the avoidance of doubt, therefore, although the following examples focus on use of a smartwatch as the first apparatus, the first apparatus can be another type of apparatus.

The one or more second sensors may be of any suitable type.

For example, the one or more second sensors may be configured to sense one or more of heart rate, blood pressure, ECG activity, temperature and blood oxygen saturation. This list is non-exhaustive. The second sensors may comprise electrical sensors and/or chemical sensors. For example, for heart rate and/or electrocardiogram activity, the patch may comprise two ECG electrodes. For blood pressure, the patch may comprise two ultrasound transducers for sensing echoes from emitted ultrasounds which may be translated into blood pressure readings. A temperature sensor may detect a current temperature. Light sensors may be used to detect blood oxygen saturation and/or heart rate activity, for example using photoplethysmogrpahy (PPG) sensors.

The sensed second biosignals may be converted into data form as second biosignal data.

The receiver of the first apparatus may comprise any suitable receiver or transceiver, configured to receive signals transmitted from the patch. The receiver may therefore comprise a radio frequency identification (RFID) receiver and/or a Bluetooth and/or Zigbee receiver. This list is non-exhaustive.

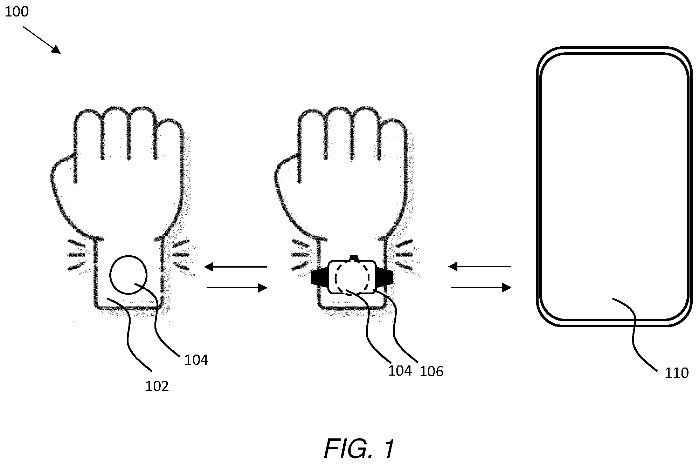

FIG. 1 shows an example system 100 according to one or more example embodiments.

The system 100 may comprise a patch 104 for placement on a user's wrist 102, or other body part. The patch 104 may comprise an adhesive surface on one side thereof which may include a hydrogel material.

The system 100 may also comprise a first apparatus, in this case a smartwatch 106 for being worn or placed over the patch 104. This may mean that the smartwatch 106 is adjacent to, and in direct contact with, the patch 104 when worn. Optionally, the system 100 may also comprise a second apparatus, in this case a smartphone 110, which may be used in conjunction with the smartwatch 106 if it is paired to it.

The smartwatch 106 and/or smartphone 110 may comprise a health monitoring application for displaying biosignal measurements.

The health monitoring application may also perform certain health monitoring analytics, such as to determine a state of a user's health based on a combination of historical and/or current biosignal data measurements. For example, based on a user's movements and heart rate, the health monitoring application may determine that the user is sleeping. If, for example, it is determined that the user is sleeping below a recommended period based on their age, a recommendation may be issued to get more sleep. More sophisticated health determinations, e.g. based on a larger number of biosignal measurements and/or data from other sources, may be performed using one or more ML models which may be trained to detect certain health conditions and possibly to make recommendations. Such ML models may be provided on the smartwatch 106 for in-situ processing. However, one or more ML models, whether the same or different to those on the smartwatch 106, may be provided on the smartphone 110. For example, the smartphone 110 may have greater processing and/or memory capabilities than the smartwatch 106, in which case some or all ML processing tasks may be offloaded to the smartphone when connected to the smartwatch. It may be that certain ML processing tasks can only be performed at the smartwatch 106 at certain times. For example, if a charge level of a battery of the smartwatch 106 is below a predetermined threshold and/or if available processing or memory resources of the smartwatch are below a predetermined threshold, then first and/or second biosignal data may be transmitted to the smartphone 110 for processing thereat. Results, e.g. health statuses and recommendations, may be displayed by the health-monitoring application of the smartphone 110 and/or may be sent back to the smartwatch 106 for display thereat.

The smartphone 110 may be configured to communicate with the smartwatch 106 using any suitable communications protocol, e.g. Bluetooth, Zigbee, WiFi, and/or any cellular communications protocol (e.g. 3G, 4G, 5G), for the transfer of data. This list is non-exhaustive. For this purpose, the smartphone 100 may be required to "pair" with the smartwatch 106 using, for example, a conventional Bluetooth pairing protocol or similar.

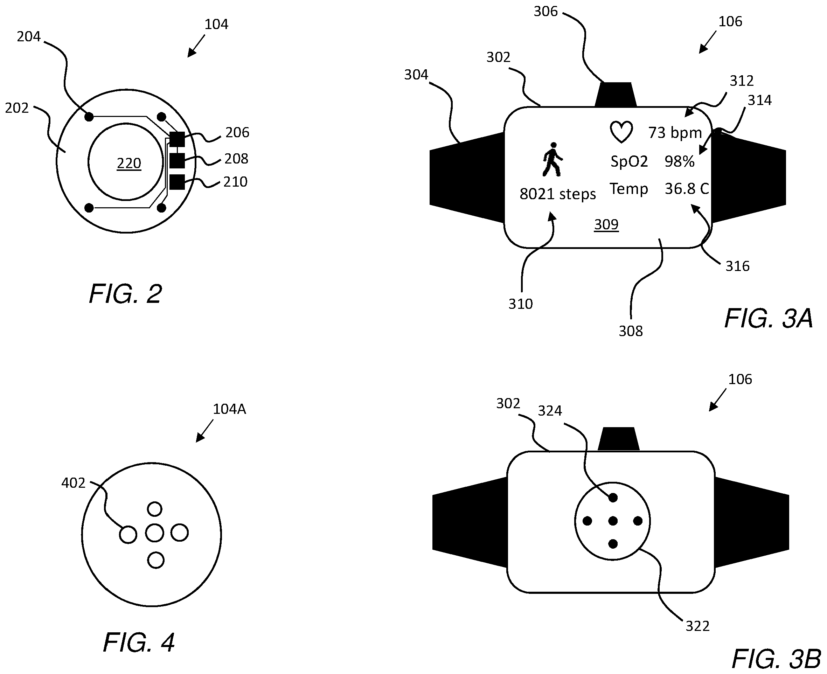

FIG. 2 shows the patch 104 in more detail.

The patch 104 may comprise a body 202 formed of a relatively thin and flexible material having, on one side thereof, an adhesive substance. The flexible material may comprise, for example, a woven fabric, plastic or latex. The patch 104 may also comprise one or more first sensors 204, a memory 206 for storing sensed first biosignal data representing the biosignals from one or more of the one or more first sensors 204, a transceiver 208 for transmitting and receiving signals to and from the smartwatch 106 and a rechargeable battery 210. Although not indicated, the rechargeable battery 210 may provide power to the one or more first sensors 204, if electric sensors, and also to the memory 206 and transceiver 208. The transceiver 208 may be connected to an antenna (not shown) which may be a patch-antenna.

The body 202 of the patch 104 may be substantially ring-shaped. That is, the body 202 may comprise an aperture 220. The aperture 220 may be substantially central on the patch 104. The aperture 220 and its location may be configured, with appropriate user-placement, to align with location(s) of one or more second sensors of the smartwatch 106, or a portion of the smartwatch that carries the one or more second sensors, when the smartwatch is worn or located over the patch 104. Put another way, the body 202 of the patch 104 may be complementary to the location(s) of the one or more second sensors. This ensures that the user's skin can be exposed to the one or more second sensors. In this respect, it will be appreciated that the patch 104 can have alternative shapes and forms and is not necessarily ring-shaped or even circular so long as the one or more second sensors are not blocked from the user's skin by the patch. For example, the perimeter of the patch may be square or rectangular in shape. For example, the aperture 220 may be square or rectangular in shape, or another shape and need not have the same shape as the patch perimeter. If ring-shaped, the ring need not be a closed ring, i.e. there may be one or more gaps. The patch may have the form of an arc or a portion of another shape that, in use, surrounds, or at least partially surrounds, the area comprising the one or more second sensors.

FIG. 3A shows the smartwatch 106 in front plan view. FIG. 3B shows the smartwatch in rear plan view.

The smartwatch 106 may comprise a main body 302, a wrist strap or band 304, an optional control button 306 and a screen 308, which may or may not be touch-sensitive. As shown, the screen 308 may display a graphical user interface (GUI) 309 associated with a health-monitoring application running on the smartwatch 106. The GUI 309 may indicate one or more biosignal data measurements, at least some of which may be derived from respective biosignals measured by second sensors 324 on the rear side. For example, the GUI 309 may display an indication 310 of a current number of steps taken, in the manner of a pedometer. For example, the GUI 309 may also display a current heart rate 312, a current blood oxygen saturation 314 and a current temperature 316 based on biosignals measured via the second sensors 324 on the rear side.

As shown in FIG. 3B, the body 302 may carry the plurality of second sensors 324 on its rear side. The second sensors 324 may be electrical sensors. The second sensors 324 may be provided on a substantially circular sensor region 322 that may be aligned with the aperture 220 of the patch 104 when the smartwatch 106 is worn or placed over the patch. The size and shape of the sensor region 322 may substantially match that of the aperture 220 or it may be smaller than the aperture. The user may be able to sense when the sensor region 322 locates within the inner circumference or boundary of the aperture 220.

As shown in FIG. 4, an alternative form of patch 104A may comprise a plurality of smaller apertures 402 (or recesses which are cut-in from the edge) which respectively align with each of the second sensors 324. Other arrangements of apertures and/or recesses may be used for achieving the same purpose.

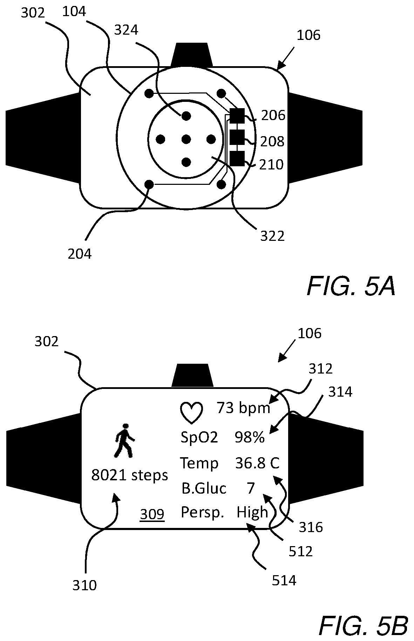

FIG. 5A shows the smartwatch 106 from the rear side when overlaid onto the FIG. 2 patch 104. The sensor region 322 is aligned with the aperture 220 of the patch 104. In this way, there is no barrier between the second sensors 324 and the user's skin. Also, the transceiver 208 and rechargeable battery 210 of the patch 104 are positioned close to the smartwatch 106 for efficient data transfer and recharging, if required. In this respect, the smartwatch 106 may be configured to emit a wireless signal for recharging the rechargeable battery 210 when the first apparatus is worn or placed over the patch 104. The smartwatch 106, when worn, helps retain the patch 104 in contact with the user's skin.

In the FIG. 5A configuration, first biosignal data measured by the first sensors 204 may be transmitted by the transceiver 208 of the patch 104 to a receiver of the smartwatch 106 using any suitable communications protocol, examples of which are given above.

Transmission by the transceiver 208 may be responsive to the smartwatch 106 detecting the patch 104 being in proximity and/or the user selecting via some input means to request first biosignal data from the patch. The smartwatch 106 may, for example, transmit an interrogation message that can be received by the transceiver 208 of the patch 104. The smartwatch 106 may transmit the interrogation message responsive to detecting that the patch 104 is in proximity to the smartwatch. This may be by means of a proximity sensor on the smartwatch 106 and/or by means the transceiver 208 acknowledging receipt of short-range beacon signal periodically transmitted by the smartwatch. The transceiver 208 of the patch 104 may then automatically respond, with transmission of the first biosignal data. This may be by means of, for example, a radiofrequency identifier (RFID) or Bluetooth responsive message. The first biosignal data may represent real-time biosignal measurements and/or past biosignal measurements stored in the memory 206. Such past biosignal measurements may be associated with respective timestamps indicative of when the measurements were recorded, e.g. in terms of dates and times or offsets from a reference time. For example, it will be appreciated that first biosignal data may be generated and stored in the memory 206 of the patch 104 at times when the user is not wearing the smartwatch 106, as in the first mode mentioned above. Hence, the health-monitoring application on the smartwatch 106 can obtain backdated first biosignal data and provide a more meaningful and comprehensive analysis of the user's health status.

FIG. 5B shows the smartwatch 106, again in front plan view, when overlaid onto the patch 104. It may be assumed that the health-monitoring application on the smartwatch 106 may analyse and/or display both the first and second biosignal data, the former being received from the patch 104. As will be seen, the GUI 309 of the health-monitoring application may now display (in addition to measurements of the number of steps taken 310, heart rate 312, blood oxygen saturation 314, and temperature 316) measurement of blood glucose level 512 and perspiration level 514. This combination of measurements may represent a default combination; the user may have the option to change which measurements are shown on the GUI 309.

In accordance with example embodiments, the FIG. 1 system may operate in at least three modes.

In a first mode, the user may only be wearing the patch 104. There may be situations where the user prefers not to wear the smartwatch 106, e.g. when they are sleeping, swimming or performing some other form of exercise or activity. In the first mode, the first sensors 204 may measure first biosignals and store resulting first biosignal data in the memory 206 with, for example, metadata indicative of the respective sensor(s) 204 from which the data is received and associated timestamps.

In a second mode, the user may only be wearing the smartwatch 106. In the second mode, the second sensors 324 may measure second biosignals and store resulting second biosignal data. The second biosignal data may be stored on an on-board memory of the smartwatch 106 with, for example, metadata indicative of the respective sensor(s) 324 from which the data is received and associated timestamps. The smartwatch 106 may run the health-monitoring application with GUI 309 for displaying the default or selected combination of biosignal measurements.

In a third mode, the user may be wearing both the patch 104 and the smartwatch 106 in the manner shown in FIG. 5B. The third mode may be considered a co-operative mode, in that the smartwatch may receive from the patch 104 the first biosignal data, which may be current data, historic data or both current and historic data. In the third mode, the smartwatch 106 may therefore analyse and/or display both the first and second biosignal data. The user may also have the option to select which of the first and/or second biosignal data is analysed and/or displayed. For example, the user may only wish to have the first biosignal data or a particular combination of first and second biosignal data to be analysed and/or displayed.

It is to be noted that the patch 104 and smartwatch 106 may comprise respective first and second sensors for measuring one or more common biosignals, i.e. biosignals of the same type e.g. heart rate. In the first mode, therefore, the patch 104 is capable of measuring and storing heart rate values for the user over a first time period. In the second mode, the smartwatch 106 may also measure and store heart rate values for the user over a different, second time period. In the third mode, the smartwatch 106 may receive the heart rate values for the first time period (from the memory of 206 the patch 104) and may combine with the heart rate values for the second time period from the smartwatch to obtain a more complete temporal history.

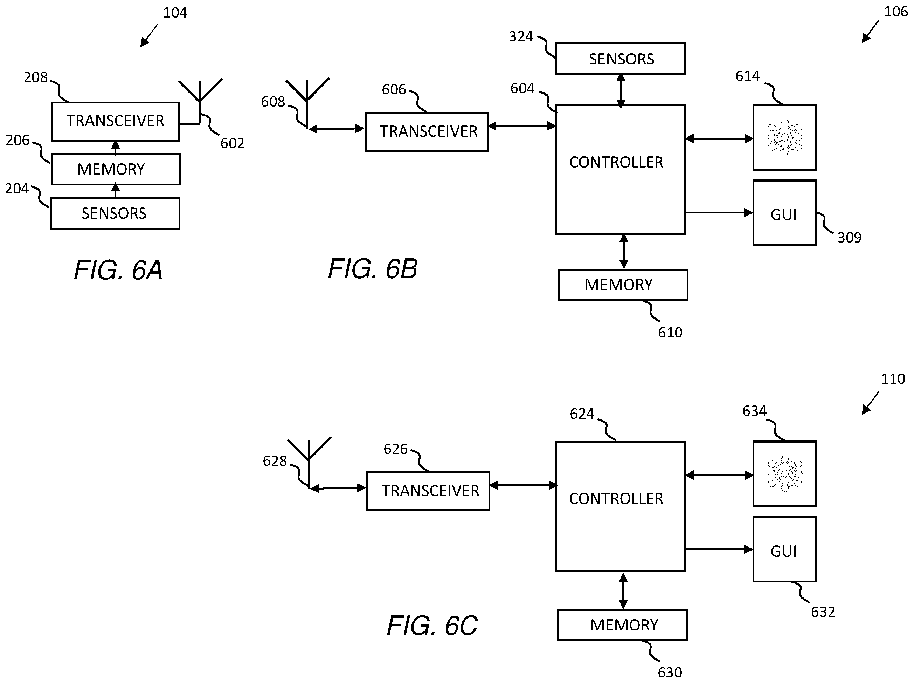

FIG. 6A is a schematic view of functional components of the patch 104. The patch 104 may comprise one or more first sensors 204, a memory 206 and a transceiver 208. The memory 206 may comprise any suitable form of data storage. The transceiver 208 may be connected to an antenna 602 for transmitting the first sensor data to the smartwatch 106. In some example embodiments, the patch 104 comprises little or no processing capability. It is therefore relatively simple and cheap to produce.

FIG. 6B is a schematic view of functional components of the smartwatch 106. The smartwatch 106 may comprise a controller 604, the one or more second sensors 324, and a transceiver 606 which may be connected to an antenna 608. The controller 604 may comprise one or more microcontrollers and/or one or more microprocessors. The smartwatch 106 may also comprise a memory 610 on which first and/or second biosignal data may be stored. The memory 610 may be of any suitable data storage type, e.g. a hard disk drive (HDD) or solid state drive (SSD). The memory 610 may also store one or more applications comprising computer-readable instructions which may be run by the controller 604. For example, the above-mentioned health-monitoring application may be stored on the memory 610 for being run by the controller 604. The smartwatch 106 may also provide, via the health-monitoring application, the above-mentioned GUI 309. The smartwatch 106 may also provide one or more ML models trained to perform analysis on one or both of the first and second biosignal data, the outputs of which may be displayed by the GUI 309.

FIG. 6C is a schematic view of functional components of the optional smartphone 110. The smartphone 110 may comprise a controller 624 and a transceiver 626 which may be connected to an antenna 628. The controller 624 may comprise one or more microcontrollers and/or one or more microprocessors. The smartphone 110 may also comprise a memory 630 on which the first and/or second biosignal data may be stored, if received from the smartwatch 106. The memory 630 may be of any suitable data storage type, e.g. a hard disk drive (HDD) or solid state drive (SSD). The memory 630 may also store one or more applications comprising computer-readable instructions that may be run by the controller 624. For example, the smartphone 110 may also provide a health-monitoring application, which may be the same or similar to that provided on the smartwatch 106. The smartphone 110 may also provide, via its health-monitoring application, a GUI 632. The smartphone 110 may also provide one or more ML models trained to perform analysis on one or both of the first and second biosignal data the outputs of which may be displayed by the GUI 632 and/or which may be transmitted back to the smartwatch 106 using the transceiver 626.

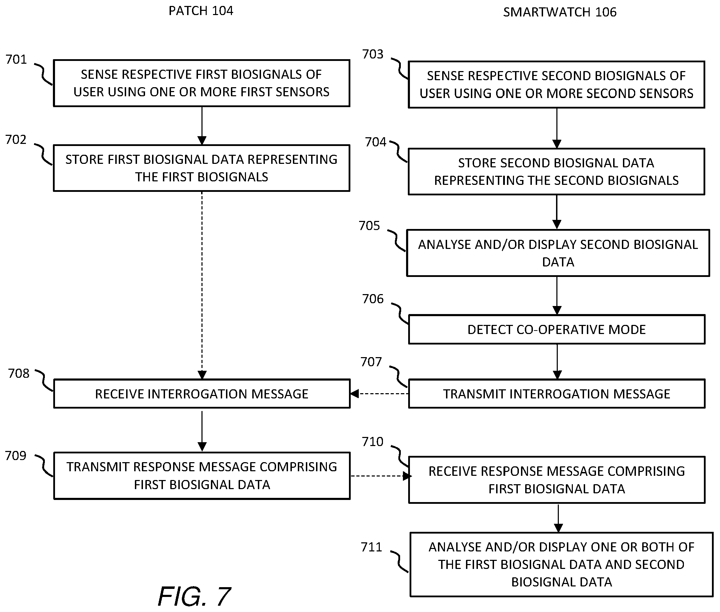

FIG. 7 is a flow diagram showing operations that may be performed by the patch 104 and smartwatch 106. The operations may be performed using hardware, software, firmware or a combination thereof.

At the patch 104, a first operation 701 may comprise sensing respective first biosignals of a user using one or more first sensors. A second operation 702 may comprise storing first biosignal data representing the first biosignals. This corresponds to the first mode of operation.

At the smartwatch 106, a third operation 703 may comprise sensing respective second biosignals of the user using one or more second sensors. A fourth operation 704 may comprise storing second biosignal data representing the second biosignals. A fifth operation 705 may comprise analysing and/or displaying the second biosignal data. This corresponds to the second mode of operation.

A sixth operation 706 may comprise detecting that the smartwatch 106 is to operate in the third, co-operative mode of operation with the patch 104. This may be due to proximity of the patch 104 and/or user selection via the smartwatch 106 and/or another device in communication with the smartwatch, e.g. the smartphone 110. In response, a seventh operation 707 may comprise transmitting an interrogation message.

At the patch 104, an eighth operation 708 may comprise receiving the interrogation message from the smartwatch 106. A ninth operation 709 may comprise transmitting a response message comprising the first biosignal data, which can be the stored first biosignal data and/or real-time first biosignal data currently being sensed.

At the smartwatch 106, a tenth operation 710 may comprise receiving the response message comprising the first biosignal data. An eleventh operation 711 may comprise analysing and/or displaying one or both of the first biosignal data and the second biosignal data.

Although not shown in FIG. 7, further operations may comprise, at the smartwatch 106, detecting that processing and/or memory resources and/or battery resources are insufficient for analysing one or both of the first biosignal data and the second biosignal data. These may be ML based analysis operations which can require significant resources. Responsive to said detection, the smartwatch 106 may determine if it is paired with the smartphone 110 and, if so, transmits to the smartphone 110 the first biosignal data and/or the second biosignal data. At the smartphone 110, the first biosignal data and/or the second biosignal data may be received and analysed, e.g. using one or more ML models. Analysis results may be displayed on a GUI of the smartphone 110 and/or transmitted back to the smartwatch 106 for display.

Example embodiments provide a system for cooperative sensing and sharing of biosignal data for more comprehensive display and analysis, taking into account that users may prefer not to wear, for example, a smartwatch all of the time. Example embodiments provide a patch and apparatus system in which the apparatus helps retain the patch on the user whilst also allowing sensors to be exposed to the user's skin.

### Example Apparatus

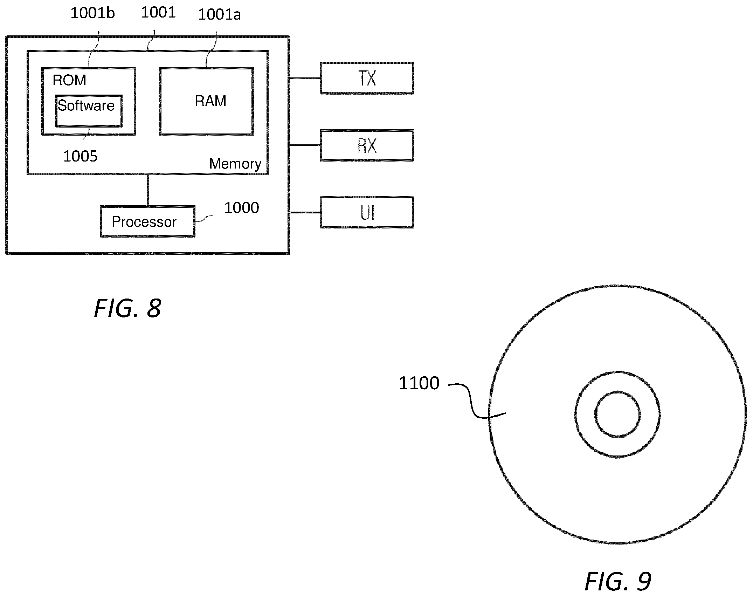

FIG. 8 shows an apparatus according to some example embodiments. The apparatus may be configured to perform operations described herein, for example operations associated with the patch 104, the smartwatch 106 and/or the smartphone 110. The apparatus comprises at least one processor 1000 and at least one memory 1001 directly or closely connected to the processor. The memory 1001 includes at least one random access memory (RAM) 1001a and at least one read-only memory (ROM) 1001b. Computer program code (software) 1005 is stored in the ROM 1001b. The apparatus may be connected to a transmitter (TX) and a receiver (RX). The apparatus may, optionally, be connected with a user interface (UI) for instructing the apparatus and/or for outputting data. The at least one processor 1000, with the at least one memory 1001 and the computer program code 1005 are arranged to cause the apparatus to at least perform at least the method according to any preceding process.

FIG. 9 shows a non-transitory media 1100 according to some embodiments. The non-transitory media 1100 is a computer readable storage medium. It may be e.g. a CD, a DVD, a USB stick, a blue ray disk, etc. The non-transitory media 1100 stores computer program instructions, causing an apparatus to perform the method of any preceding process. As illustrated in Fig. 9 the computer program code 1005 can arrive at the apparatus via any suitable delivery mechanism. The delivery mechanism can be, for example, a machine readable medium, a computer-readable medium, a non-transitory computer-readable storage medium, a computer program product, a memory device, a record medium such as a Compact Disc Read-Only Memory (CD-ROM) or a Digital Versatile Disc (DVD) or a solid-state memory (e.g. SSD or USB stick), an article of manufacture that comprises or tangibly embodies the computer program 1005. The delivery mechanism can be a signal configured to reliably transfer the computer program 1005. The apparatus can propagate or transmit the computer program 1005 as a computer data signal. In some examples the computer program 1005 can be transmitted to the apparatus using a wireless protocol such as Bluetooth, Bluetooth Low Energy, Bluetooth Smart, 6LoWPan (IPᵥ6 over low power personal area networks) ZigBee, ANT+, near field communication (NFC), Radio frequency identification, wireless local area network (wireless LAN) or any other suitable protocol.

Names of network elements, protocols, and methods are based on current standards. In other versions or other technologies, the names of these network elements and/or protocols and/or methods may be different, as long as they provide a corresponding functionality. For example, embodiments may be deployed in 2G/3G/4G/5G networks and further generations of 3GPP but also in non-3GPP radio networks such as WiFi.

A memory may be volatile or non-volatile. It may be e.g. a RAM, a SRAM, a flash memory, a FPGA block ram, a DVD, a CD, a USB stick, and a blue ray disk.

If not otherwise stated or otherwise made clear from the context, the statement that two entities are different means that they perform different functions. It does not necessarily mean that they are based on different hardware. That is, each of the entities described in the present description may be based on a different hardware, or some or all of the entities may be based on the same hardware. It does not necessarily mean that they are based on different software. That is, each of the entities described in the present description may be based on different software, or some or all of the entities may be based on the same software. Each of the entities described in the present description may be embodied in the cloud. Implementations of any of the above described blocks, apparatuses, systems, techniques or methods include, as non-limiting examples, implementations as hardware, software, firmware, special purpose circuits or logic, general purpose hardware or controller or other computing devices, or some combination thereof. Some embodiments may be implemented in the cloud.

It is to be understood that what is described above is what is presently considered the preferred embodiments. However, it should be noted that the description of the preferred embodiments is given by way of example only and that various modifications may be made without departing from the scope of the invention, which is defined by the appended claims.

## Claims

1. A system comprising:
a patch (104) comprising:
a surface for attachment to a user;
one or more first sensors (204) for sensing respective first biosignals of the user; and
a transmitter (208) for wirelessly transmitting first biosignal data
representing the first biosignals of the user; and
an apparatus (106) for being worn or placed over the patch, the apparatus comprising:
one or more second sensors (324) at or near a lower surface thereof for sensing respective second biosignals of the user and providing second biosignal data representing the second biosignals; and
a receiver (606) for wirelessly receiving the first biosignal data from the patch;
wherein the apparatus is configured to analyse and/or display one or both of the first and second biosignal data, and
wherein the patch comprises one or more recesses or apertures (220) for alignment with the one or more second sensors when the apparatus is worn or placed over the patch so that the user's skin is exposed to the one or more second sensors, the system being **characterised in that**
at least one of the one or more first sensors is configured to sense a same type of biosignal as at least one of the one or more second sensors,
wherein, at a first time when the apparatus is located over the patch, the receiver is configured to wirelessly receive, from a memory (206) of the patch, historic first biosignal data representing said same type of biosignal , by means of which the apparatus is configured, at a second, subsequent time to the first time, to fill-in temporal gaps in the second biosignal data for, at least in part, analysis thereof.

2. The system of claim 1, wherein at least one of the one or more first sensors is configured to sense a different type of biosignal than any of the one or more second sensors; and wherein the apparatus is configured to analyse and/or display the first biosignal data and the second biosignal data comprising the different types of biosignal.

3. The system of any preceding claim, wherein the apparatus comprises a substantially circular portion comprising the one or more second sensors, and wherein the patch is substantially ring-shaped.

4. The system of any preceding claim, wherein the patch further comprises a rechargeable energy storage means for providing power to the one or more first sensors and/or the transmitter and wherein the apparatus is further configured to emit a wireless signal for recharging the energy storage means when the apparatus is worn over the patch.

5. The system of any preceding claim, wherein
the apparatus further comprises a transmitter for transmitting an interrogation message to the patch; and
the patch further comprises a memory for storing the first biosignal data, and a transmitter for wirelessly transmitting, responsive to receiving the interrogation message, the stored first biosignal data to the apparatus.

6. The system of claim 5, wherein the apparatus is configured to transmit the interrogation message responsive to detecting the patch is in proximity to the apparatus.

7. The system of any preceding claim, wherein the apparatus comprises one or more machined-learned models for performing analysis of one or both of the first biosignal data and the second biosignal data.

8. The system of claim 7, wherein the apparatus is further configured to transmit, to a remote second apparatus, one or both of the first and second biosignal data responsive to detecting a predetermined condition, for analysis by one or more machined-learned models at the second apparatus.

9. The system of claim 8, wherein the predetermined condition comprises one or more of:
a charge level of a battery of the apparatus being below a predetermined threshold; and
available processing resources of the apparatus being below a predetermined threshold.

10. The system of any preceding claim, wherein the first biosignal data represents one or more of heart rate, blood glucose level, perspiration and sodium level.

11. The system of any preceding claim, wherein the second biosignal data represents one or more of heart rate, blood pressure, electrocardiogram activity, temperature, and blood oxygen saturation.

## Patentansprüche

1. System, das Folgendes umfasst:
ein Patch (104), das Folgendes umfasst:
eine Fläche zur Befestigung an einem Benutzer;
einen oder mehrere erste Sensoren (204) zum Erfassen von jeweiligen ersten Biosignalen des Benutzers; und
einen Sender (208) zum drahtlosen Übertragen von ersten Biosignaldaten, die die ersten Biosignale des Benutzers repräsentieren; und
eine Einrichtung (106) zum Getragenwerden oder zum Platzieren auf dem Patch, wobei die Einrichtung Folgendes umfasst:
einen oder mehrere zweite Sensoren (324) an oder in der Nähe einer unteren Fläche davon zum Erfassen der jeweiligen zweiten Biosignale des Benutzers und Bereitstellen von zweiten Biosignaldaten, die die zweiten Biosignale repräsentieren; und
einen Empfänger (606) zum drahtlosen Empfangen der ersten Biosignaldaten vom Patch;
wobei die Einrichtung dazu ausgelegt ist, eines oder beides der ersten und der zweiten Biosignaldaten zu analysieren und/oder anzuzeigen, und
wobei das Patch eine oder mehrere Ausnehmungen oder Öffnungen (220) zur Ausrichtung auf den einen oder die mehreren zweiten Sensoren umfasst, wenn die Einrichtung getragen oder auf dem Patch platziert ist, derart, dass die Haut des Benutzers zu dem einen oder den mehreren zweiten Sensoren exponiert ist,
wobei das System **dadurch gekennzeichnet ist, dass** mindestens einer des einen oder der mehreren ersten Sensoren dazu ausgelegt ist, eine selbe Art Biosignal zu erfassen wie mindestens einer des einen oder der mehreren zweiten Sensoren,
wobei der Empfänger zu einer ersten Zeit, wenn sich die Einrichtung auf dem Patch befindet, dazu ausgelegt ist, historische erste Biosignaldaten, die dieselbe Art von Biosignal repräsentieren, von einem Speicher (206) des Patches drahtlos zu empfangen, mittels derer die Einrichtung dazu ausgelegt ist, zu einer zweiten, der ersten Zeit nachfolgenden Zeit zeitliche Lücken in den zweiten Biosignaldaten für mindestens teilweise eine Analyse davon zu füllen.

2. System nach Anspruch 1, wobei mindestens einer des einen oder der mehreren ersten Sensoren dazu ausgelegt ist, eine andere Art von Biosignal als einer des einen oder der mehreren zweiten Sensoren zu erfassen; und wobei die Einrichtung dazu ausgelegt ist, die ersten Biosignaldaten und die zweiten Biosignaldaten, die die verschiedenen Arten von Biosignal umfassen, zu analysieren und/oder anzuzeigen.

3. System nach einem der vorhergehenden Ansprüche, wobei die Einrichtung einen im Wesentlichen kreisförmigen Abschnitt umfasst, der den einen oder die mehreren zweiten Sensoren umfasst, und wobei das Patch im Wesentlichen ringförmig ist.

4. System nach einem der vorhergehenden Ansprüche, wobei das Patch ferner zum Bereitstellen von Strom für den einen oder die mehreren ersten Sensoren und/oder den Sender ein aufladbares Energiespeichermittel umfasst und wobei die Einrichtung ferner dazu ausgelegt ist, ein drahtloses Signal zum Aufladen des Energiespeichermittels zu emittieren, wenn die Einrichtung auf dem Patch getragen wird.

5. System nach einem der vorhergehenden Ansprüche, wobei
die Einrichtung ferner zum Übertragen einer Abfragenachricht zum Patch einen Sender umfasst; und
das Patch ferner zum Speichern der ersten Biosignaldaten einen Speicher umfasst und in Reaktion auf das Empfangen der Abfragenachricht zum drahtlosen Übertragen der gespeicherten ersten Biosignaldaten zur Einrichtung einen Sender umfasst.

6. System nach Anspruch 5, wobei die Einrichtung dazu ausgelegt ist, die Abfragenachricht in Reaktion auf das Detektieren des Patches in der Nähe der Einrichtung zu übertragen.

7. System nach einem der vorhergehenden Ansprüche, wobei die Einrichtung zum Durchführen einer Analyse von einem oder beidem der ersten Biosignaldaten und der zweiten Biosignaldaten ein oder mehrere maschinengelernte Modelle umfasst.

8. System nach Anspruch 7, wobei die Einrichtung ferner dazu ausgelegt ist, eines oder beides der ersten und der zweiten Biosignaldaten in Reaktion auf das Detektieren einer vorbestimmten Bedingung zur Analyse durch ein oder mehrere maschinengelernte Modelle an einer zweiten Einrichtung zur entfernten zweiten Einrichtung zu übertragen.

9. System nach Anspruch 8, wobei die vorbestimmte Bedingung eines oder mehreres von Folgendem umfasst:
ein Ladungsniveau einer Batterie der Einrichtung liegt unter einem vorbestimmten Schwellwert; und
verfügbare Verarbeitungsressourcen der Einrichtung liegen unter einem vorbestimmten Schwellwert.

10. System nach einem der vorhergehenden Ansprüche, wobei die ersten Biosignaldaten eines oder mehreres von einer Herzfrequenz, einem Blutglukoseniveau, einer Perspiration und eines Natriumniveaus repräsentieren.

11. System nach einem der vorhergehenden Ansprüche, wobei die zweiten Biosignaldaten eines oder mehreres von einer Herzfrequenz, einem Blutdruck, einer Elektrokardiogrammaktivität, einer Temperatur und einer Blutsauerstoffsättigung repräsentieren.

## Revendications

1. Système comprenant :
une plaque (104) comprenant :
une surface destinée à être fixée sur un utilisateur ;
un ou plusieurs premiers capteurs (204) pour détecter des premiers signaux biologiques respectifs de l'utilisateur ; et
un émetteur (208) pour transmettre sans fil des premières données de signal biologique représentant les premiers signaux biologiques de l'utilisateur ; et
un appareil (106) destiné à être porté ou placé par-dessus la plaque, l'appareil comprenant :
des deuxièmes capteurs (324) situés au niveau ou à proximité d'une surface inférieure de celui-ci pour détecter des deuxièmes signaux biologiques respectifs de l'utilisateur et fournir des deuxièmes données de signal biologique représentant les deuxièmes signaux biologiques ; et
un récepteur (606) pour recevoir sans fil les premières données de signal biologique provenant de la plaque ;
dans lequel l'appareil est configuré pour analyser et/ou afficher les premières données de signal biologique et/ou les deuxièmes données de signal biologique, et
dans lequel la plaque comprend un ou plusieurs renfoncements ou ouvertures (220) pour l'aligner avec les un ou plusieurs deuxièmes capteurs lorsque l'appareil est porté ou placé par-dessus la plaque de sorte que la peau de l'utilisateur soit exposée aux un ou plusieurs deuxièmes capteurs,
le système étant **caractérisé en ce que**
au moins un des un ou plusieurs premiers capteurs est configuré pour détecter un même type de signal biologique qu'au moins un des un ou plusieurs deuxièmes capteurs,
dans lequel, à un premier moment où l'appareil est situé par-dessus la plaque, le récepteur est configuré pour recevoir sans fil d'une mémoire (206) de la plaque des premières données de signal biologique historiques représentant ledit même type de signal biologique, grâce auxquelles l'appareil est configuré, et à un deuxième moment postérieur au premier moment, pour combler des écarts temporels dans les deuxièmes données de signal biologique pour les analyser au moins partiellement.

2. Système selon la revendication 1, dans lequel au moins un des un ou plusieurs premiers capteurs est configuré pour détecter un type de signal biologique différent de l'un quelconque des un ou plusieurs deuxièmes capteurs ; et dans lequel l'appareil est configuré pour analyser et/ou afficher les premières données de signal biologique et les deuxièmes données de signal biologique comprenant les types différents de signal biologique.

3. Système selon l'une des revendications précédentes, dans lequel l'appareil comprend une partie sensiblement circulaire comprenant les un ou plusieurs deuxièmes capteurs, et dans lequel la plaque est sensiblement en forme d'anneau.

4. Système selon l'une des revendications précédentes, dans lequel la plaque comprend en outre des moyens de stockage d'énergie rechargeable pour fournir de la puissance aux un ou plusieurs premiers capteurs et/ou à l'émetteur, et dans lequel l'appareil est en outre configuré pour émettre un signal sans fil pour recharger les moyens de stockage d'énergie lorsque l'appareil est porté par-dessus la plaque.

5. Système selon l'une des revendications précédentes, dans lequel
l'appareil comprend en outre un émetteur pour transmettre un message d'interrogation à la plaque ; et
la plaque comprend en outre une mémoire pour stocker les premières données de signal biologique, et en réponse à la réception du message d'interrogation, un émetteur pour transmettre sans fil à l'appareil les premières données de signal biologique stockées.

6. Système selon la revendication 5, dans lequel l'appareil est configuré pour transmettre le message d'interrogation en réponse à la détection de la plaque à proximité de l'appareil.

7. Système selon l'une des revendications précédentes, dans lequel l'appareil comprend un ou plusieurs modèles d'apprentissage machine pour effectuer l'analyse des premières données de signal biologique et et/ou des deuxièmes données de signal biologique.

8. Système selon la revendication 7, dans lequel l'appareil est en outre configuré pour, en réponse à la détection d'une condition prédéterminée, transmettre à un deuxième appareil distant les premières données de signal biologique et/ou les deuxièmes données de signal biologique pour les analyser par un ou plusieurs modèles d'apprentissage machine sur le deuxième appareil.

9. Système selon la revendication 8, dans lequel la condition prédéterminée comprend un ou plusieurs des cas suivants :
un niveau de charge d'une batterie de l'appareil est en dessous d'un seuil prédéterminé ; et
des ressources de traitement disponibles de l'appareil sont en dessous d'un seuil prédéterminé.

10. Système selon l'une des revendications précédentes, dans lequel les premières données de signal biologique représentent un ou plusieurs parmi la fréquence cardiaque, le taux de glycémie, le niveau de transpiration et le taux de sodium.

11. Système selon l'une des revendications précédentes, dans lequel les deuxièmes données de signal biologique représentent un ou plusieurs parmi la fréquence cardiaque, la pression artérielle, l'activité électrocardiographique, la température et la saturation en oxygène du sang.
